# EUROPEAN PATENT APPLICATION

(11) **EP 4 471 003 A1**
(43) Date of publication of application: **04.12.2024**
(21) Application number: 23820106.5
(22) Date of filing: 08.06.2023
(51) Int. Cl.: C07C 69/734, A61K 31/235, A61K 8/37, A61Q 19/00

(54) **NOVEL COMPOUND AND USE THEREOF**

(30) Priority: 08.06.2022 KR 20220069329
(71) Applicant: Activon Co., Ltd., Cheongju-si, Chungcheongbuk-do 28104 (KR)
(72) Inventor: JEON, Ju Hee, Cheongju-si Chungcheongbuk-do 28113 (KR); JUNG, Sung Won, Yongin-si Gyeonggi-do 16909 (KR); CHO, Se Hee, Seoul 07691 (KR); PARK, Byung Gyu, Suwon-si Gyeonggi-do 16668 (KR); CHO, Youn Ki, Yongin-si Gyeonggi-do 17005 (KR); RYU, Hyung Chul, Hwaseong-si Gyeonggi-do 18526 (KR)
(74) Representative: Isarpatent
(86) International application number: PCT/KR2023/007836
(87) International publication number: WO 2023/239171

(57) **Abstract**

The present invention relates to novel compounds exhibiting excellent antioxidant effects, and cosmetic and pharmaceutical compositions including the same. The novel compounds according to the present invention are represented by Chemical Formula 1. 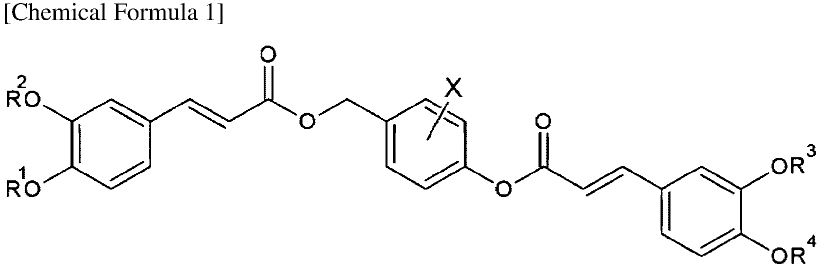 In the formula,
R¹ to R⁴ are each independently hydrogen, C₁-C₁₂ alkyl, acyl, or cyclic alkyl; and
X is hydrogen, fluoro, chloro, methyl, or methoxy.

## Description

### TECHNICAL FIELD

### Cross-references to related applications.

This application claims the benefit of priority based on Korean Patent Application No. 10-2022-0069329 filed on June 8, 2022, and the entire contents of the Korean patent application are incorporated herein by reference.

The present invention relates to novel compounds having antioxidant activities and uses thereof.

### BACKGROUND ART

The skin is the largest organ in the body and serves as a protective barrier that constantly interacts with the external environment, shielding the body from various stimuli and dry environments, and is the organ where new cells are produced and destroyed more actively compared to other organs. In addition, the skin plays a crucial role in protecting the body from physical abrasions, preventing moisture loss, shielding from ultraviolet rays emitted by the sun, and regulating body temperature.

However, in recent years, there has been an increase in factors that threaten skin health, such as increased amounts of ultraviolet radiation reaching the earth's surface due to environmental pollution, exposure to environmental pollutants such as particulate matter, westernized lifestyles, and stress. These factors not only cause skin to become dirty, dry, and troubled, but also lead to abnormalities in the skin's immune system, ultimately resulting in dermatitis, skin keratinization (roughness), and rapid aging. In particular, environmental pollution and various stresses increase the concentration of reactive oxygen species in the body, and these reactive oxygen species attack cellular components such as lipids, proteins, sugars, and DNA, causing peroxidation reactions and accelerating skin aging. Therefore, there is still a need for ingredients that provide effective defense against skin irritants generated by environmental pollution and various stresses and have excellent antioxidant capacity.

Accordingly, the inventors of the present invention have conducted diligent research to discover novel compounds that are safe and exhibit enhanced antioxidant and anti-aging effects, and as a result, they have confirmed that the compound represented by Chemical Formula 1 not only possesses excellent antioxidant activities but also demonstrates formulation stability, thereby completing the present invention.

### DISCLOSURE OF THE INVENTION

### TECHNICAL PROBLEM

One object of the present invention is to provide a novel compound or a pharmaceutically acceptable salt thereof, which exhibits excellent antioxidant activities.

Another object of the present invention is to provide a method for preparing the aforementioned novel compound.

Yet another object of the present invention is to provide a pharmaceutical composition including the compound according to the present invention or a pharmaceutically acceptable salt thereof as an active ingredient.

Still another object of the present invention is to provide a cosmetic composition including the compound according to the present invention or a cosmetologically acceptable salt thereof as an active ingredient.

### TECHNICAL SOLUTION

As one aspect of achieving the aforementioned objects, the present invention provides a compound represented by the following Chemical Formula 1, an isomer thereof, or a pharmaceutically acceptable salt thereof.

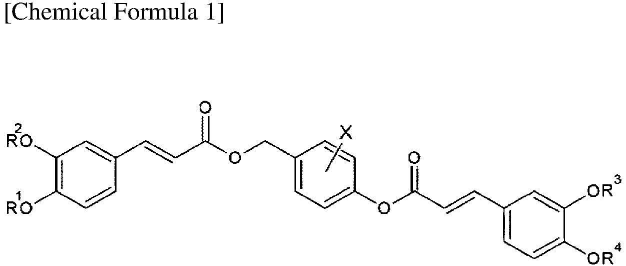

In the above formula,
R¹ to R⁴ are independently hydrogen, C₁-C₁₂ alkyl, preferably C₁-C₆ alkyl, more preferably C₁-C₃ alkyl, C₁-C₁₂ acyl, preferably C₁-C₆ acyl, more preferably C₁-C₃ acyl, or C₃-C₁₂ cyclic alkyl, preferably C₃-C₁₀ cyclic alkyl, more preferably C₃-C₆ cyclic alkyl, and
X is hydrogen, fluoro, chloro, methyl, or methoxy.

More specifically, the compound represented by Chemical Formula 1 according to the present invention may be selected from a group consisting of compounds having the following structures.

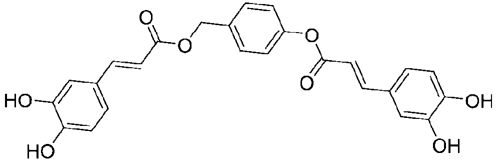

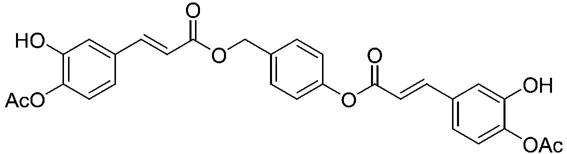

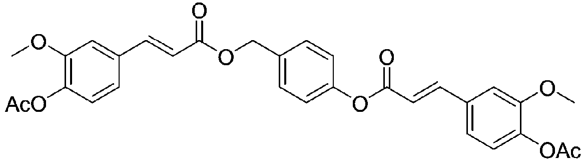

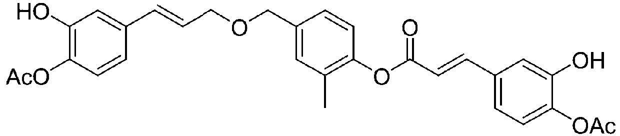

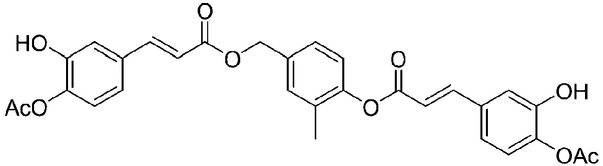

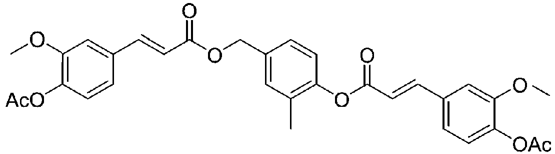

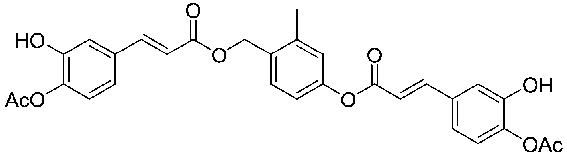

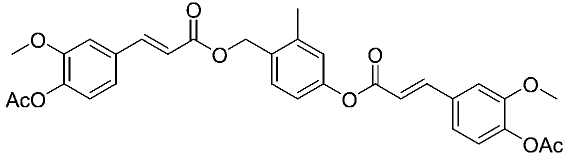

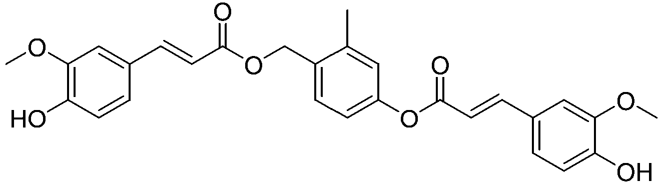

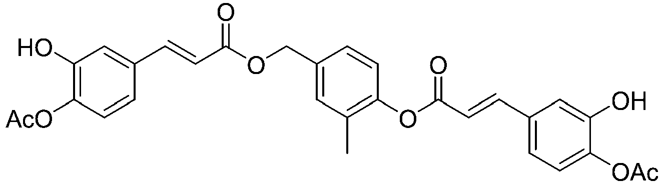

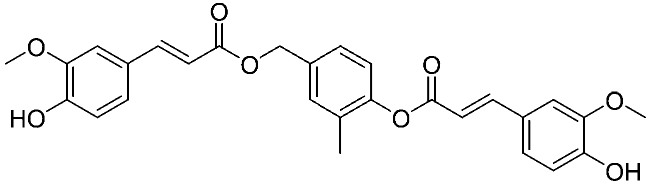

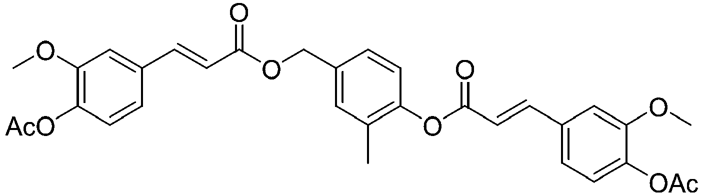

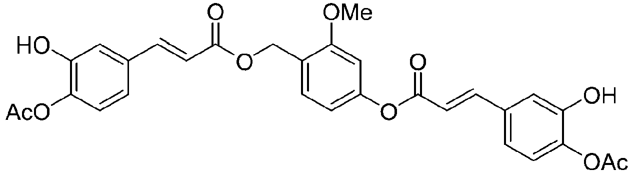

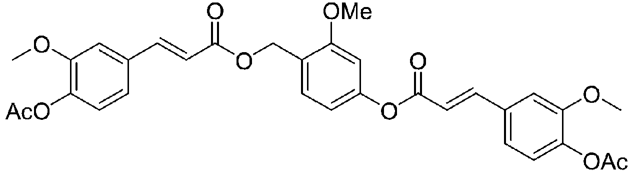

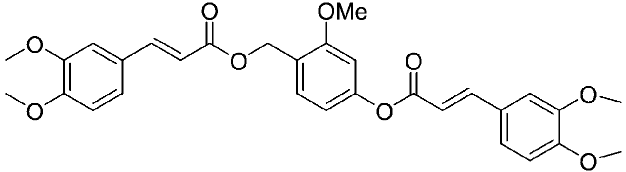

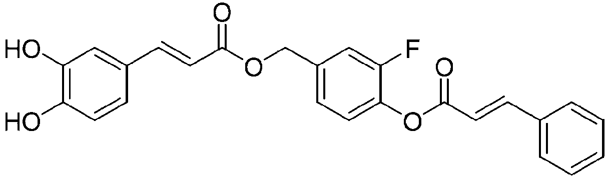

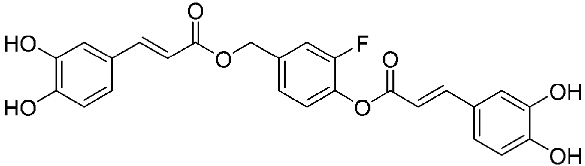

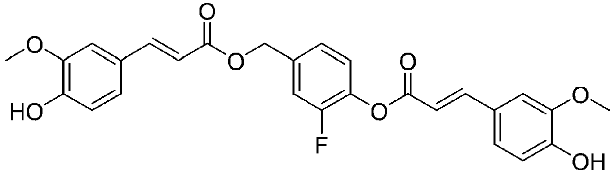

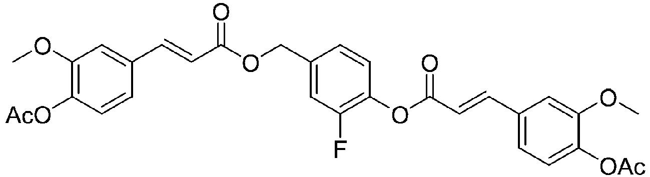

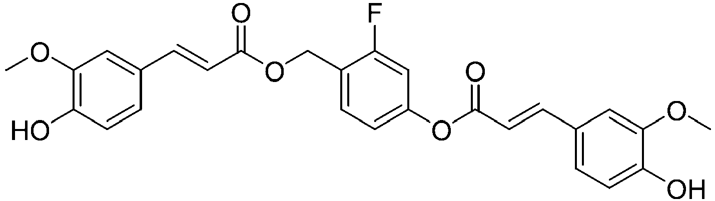

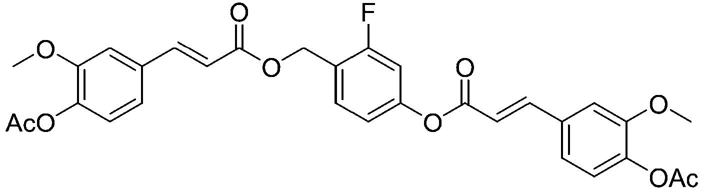

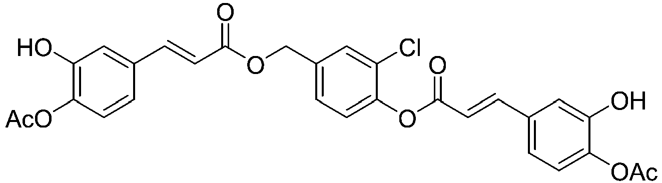

Even more specifically, the compound represented by Chemical Formula 1 according to the present invention may be selected from a group consisting of the following compounds.
4-(((E)-3-(3,4-dihydroxyphenyl)acryloyl)oxy)benzyl-(E)-3-(3,4-dihydroxyphenyl)acrylate;
(E)-4-(((E)-3-(4-acetoxy-3-hydroxyphenyl)acryloyl)oxy)benzyl-3-(4-acetoxy-3-hydroxyphenyl)acrylate;
(E)-4-(((E)-3-(4-acetoxy-3-hydroxyphenyl)acryloyl)oxy)-benzyl-3-(4-acetoxy-3-hydroxyphnyl)acrylate;
(E)-4-(((E)-3-(4-acetoxy-3-hydroxyphenyl)acryloyl)oxy)-2-methylbenzyl-3-(4-acetoxy-3-hydroxyphenyl)acrylate;
(E)-4-(((E)-3-(4-acetoxy-3-methoxyphenyl)acryloyl)oxy)-3-methylbenzyl-3-(4-acetoxy-3-methoxyphenyl) acrylate;
(E)-4-(((E)-3-(4-acetoxy-3-hydroxyphenyl)acryloyl)oxy)-3-methylbenzyl-3-(4-acetoxy-3-hydroxyphenyl)acrylate;
(E)-4-(((E)-3-(4-acetoxy-3-methoxyphenyl)acryloyl)oxy)benzyl-3-(4-acetoxy-3-methoxyphenyl) acrylate;
(E)-4-(((E)-3-(4-acetoxy-3-methoxyphenyl)acrloyl)oxy)-3-methybenzyl-3-(4-acetoxy-3-methoxyphenyl) acrylate;
(E)-4-(((E)-3-(4-acetoxy-3-hydroxyphenyl)acryloyl)oxy-2-methylbenzyl-3-(4-acetoxy-3-hydroxyphenyl)acryalate;
(E)-4-(((E)-3-(4-acetoxy-3-hydroxyphenyl)acryloyl)oxy)-2-methoxybenzyl-3-(4-acetoxy-3-hydroxyphenyl)acrylate;
(E)-4-(((E)-3-(4-hydroxy-3-methoxyphenyl)acryloyl)oxy)-2-methylbenzyl-3-(4-hydroxy-3-methoxyphenyl)acrylate;
(E)-4-(((E)-3-(4-hydroxy-3-methoxyphenyl)acryloyl)oxy)-3-methylbenzyl-3-(4-hydroxy-3-methoxyphenyl)acrylate;
(E)-4-(((E)-3-(4-acetoxy-3-methoxyphenyl)acryloyl)oxy)-2-methoxybenzyl-3-(4-acetoxy-3-methoxyphenyl)acrylate;
(E)-4-(((E)-3-(4-acetoxy-3-methoxyphenyl)acryloyl)oxy)-2-methylbenzly-3-(4-acetoxy-3-methoxyphenyl)acrylate; (E)-4-(((E)-3-(4-acetoxy-3-hydroxyphenyl)acryloyl)oxy)benzyl-3-(4-acetoxy-3-hydroxyphenyl)acrylate;
(E)-4-(((E)-3-(4-hydroxy-3-methoxyphenyl)acryloyl)oxy)-3-methybenzly-3-(4-hydroxy-3-methoxyphenyl) acrylate;
(E)-4-(((E)-3-(4-hydroxy-3-methoxyphenyl)acryloyl)oxy)-2-methybenzly-3-(4-hydroxy-3-methoxyphenyl) acrylate;
(E)-4-(((E)-3-(3,4-dimethoxyphenyl)acryloyl)oxy)-2-methoxybenzyl-3-(3,4-dimethoxyphenyl) acrylate;
(E)-4-(((E)-3-(4-acetoxy-3-methoxyphenyl)acryloyl)oxy)-2-methylbenzyl-3-(4-acetoxy-3-methoxyphenyl) acrylate;
(E)-4-(((E)-3-(3,4-dihydroxyphenyl)acryloyl)oxy)-3-fluorobenzyl-3-(3,4-dihydroxyphenyl)acrylate;
(E)-4-(((E)-3-(4-acetoxy-3-methoxyphenyl)acryloyl)oxy)-3-fluorobenzyl-3-(4-acetoxy-3-methoxyphenyl) acrylate;
((E)-2-fluoro-4-((((E)-3-(4-hydroxy-3-methoxyphenyl)acryloyl)oxy)methyl)phenyl-3-(4-hydroxy-3-methoxyphenyl)acrylate;
(E)-4-(((E)-3-(4-acetoxy-3-hydroxyphenyl)acryloyl)oxy)-3-chlorobenzyl-3-(4-acetoxy-3-hydroxyphenyl)acrylate;
(E)-2-fluroro-((((E)-3-(4-hydroxy-3-methoxyphenyl)acryloyl)oxy)methyl)phenyl-3-(4-hydroxy-3-methoxyphenyl)acrylate;
(E)-3-fluoro-4-((((E)-3-(4-hydroxy-3-methoxyphenyl)acryloyl)oxy)methyl)phenyl-3-(4-hydroxy-3-methoxyphenyl)acrylate;
(E)-2-fluoro-4-((((E)-3-(4-hydroxy-3-methoxyphenyl)acryloyl)oxy)methyl)phenyl-3-(4-hydroxy-3-methoxyphenyl)acrylate and
(E)-4-(((E)-3-(4-acetoxy-3-methoxyphenyl)acryloyl)oxy)-2-fluorobenzyl-3-(4-acetoxy-3-methoxyphenyl) acrylate.

In addition, the present invention provides a method of preparing the compound represented by Chemical Formula 1, including the step of reacting (E)-3-(3,4-dihydroxyphenyl) acrylic acid as a starting material.

In another aspect, the present invention provides a pharmaceutical composition including, as an active ingredient, one or more of the compounds represented by Chemical Formula 1, an isomer thereof, or a pharmaceutically acceptable salt thereof. The pharmaceutical compositions according to the present invention are suitable for providing an antioxidant effect to a subject.

In addition, the compound according to the present invention may be used for preventing or treating diseases caused or mediated by oxidative stress in vivo, particularly reactive oxygen species (ROS). Such diseases include, but not limited to, degenerative neurological diseases including atherosclerosis, Lou Gehrig's disease, Parkinson's disease, Alzheimer's disease, amyotrophic lateral sclerosis, and Huntington's disease; cardiovascular diseases including myocardial infarction, angina pectoris, coronary artery disease, and ischemic heart disease; ischemic brain diseases including stroke; digestive system diseases including diabetes, gastritis, and gastric cancer; various diseases such as cancer, leukemia, aging, rheumatoid arthritis, hepatitis, and atopic dermatitis; and specifically, aging caused by reactive oxygen species.

As used herein, the term "prevention" refers to any acts of inhibiting or delaying the occurrence, spread, and recurrence of diseases caused or mediated by oxidative stress in vivo, particularly reactive oxygen species (ROS), through the administration of the compound of the present invention or a composition comprising the same, and the term "treatment" refers to any acts of ameliorating or beneficially altering the symptoms of the aforementioned diseases through the administration of the composition of the present invention.

The composition of the present invention may be formulated in various forms for use according to each intended purpose by conventional methods, such as powders, granules, tablets, capsules, suspensions, emulsions, syrups, aerosols, and other oral formulations, as well as sterile injection solutions, and may be administered orally or through various routes including intravenous, intraperitoneal, subcutaneous, rectal, and topical administration, etc. Examples of suitable carriers, excipients, or diluents that may be included in the composition include lactose, dextrose, sucrose, sorbitol, mannitol, xylitol, erythritol, maltitol, starch, acacia gum, alginate, gelatin, calcium phosphate, calcium silicate, cellulose, methylcellulose, microcrystalline cellulose, polyvinylpyrrolidone, water, methyl hydroxybenzoate, propyl hydroxybenzoate, talc, magnesium stearate, and mineral oil. In addition, the composition of the present invention may further include fillers, anti-caking agents, lubricants, wetting agents, fragrances, emulsifiers, preservatives, etc.

Solid formulations for oral administration include tablets, pills, powders, granules, capsules, etc., and these solid formulations are prepared by mixing the composition with at least one excipient, such as starch, calcium carbonate, sucrose, lactose, gelatin, etc. In addition to simple excipients, lubricants such as magnesium stearate and talc may be used.

Examples of liquid formulations for oral administration include suspensions, solutions, emulsions, and syrups, etc., and in addition to commonly used simple diluents such as water and liquid paraffin, various excipients such as wetting agents, sweeteners, flavoring agents, and preservatives may be included.

Formulations for parenteral administration include sterile aqueous solutions, non-aqueous solvents, suspensions, emulsions, lyophilized formulations, and suppositories.

Non-aqueous solvents and suspensions may include propylene glycol, polyethylene glycol, vegetable oils such as olive oil, and injectable esters such as ethyl oleate. Bases for suppositories may include Witepsol, macrogol, Tween 61, cacao butter, laurin butter, and glycerinated gelatin. Meanwhile, injections may include conventional additives such as solubilizers, tonicity agents, suspending agents, emulsifiers, stabilizers, and preservatives.

The aforementioned formulations may be prepared by conventional mixing, granulation, or coating methods and may contain the compound according to the present invention in an amount ranging from about 0.1 to 75 wt%, preferably from about 1 to 50 wt%. A unit formulation for a mammal weighing from about 50 to 70 kg may contain the compound according to the present invention in an amount of from about 10 to 200 mg.

The composition of the present invention is administered in a pharmaceutically effective amount. As used herein, the term "pharmaceutically effective amount" refers to an amount sufficient to treat a disease at a reasonable benefit/risk ratio applicable to medical treatment, without causing side effects, and the effective dosage level may be determined depending on various factors including the patient's health condition, type and severity of the disease, drug activity, sensitivity to the drug, administration method, administration time, administration route and excretion rate, duration of treatment, combined or concomitant medications, and other factors well known in the medical field. The composition of the present invention may be administered as a single therapeutic agent or in combination with other therapeutic agents, sequentially or simultaneously with conventional therapeutic agents, and may be administered as a single dose or multiple doses. Considering all the aforementioned factors, it is important to administer the minimum amount for maximum effect without side effects, which may be easily determined by the person skilled in the art.

The preferred dosage of the compound of the present invention may vary depending on the patient's condition and weight, the severity of the disease, the form of the drug, the route of administration, and the duration of administration, but may be appropriately selected by the person skilled in the art. However, to achieve the desired effect, when administered to a human subject, it may be appropriate to administer the compound of the present invention in an amount of 0.0001 to 100 mg/kg (body weight) per day, preferably 0.001 to 100 mg/kg (body weight) per day. The administration may be done once a day or divided into multiple doses through oral or parenteral routes.

Furthermore, the present invention provides a method for preventing or treating diseases caused or mediated by reactive oxygen species, including the step of administering the pharmaceutical composition to a subject in need thereof.

As used herein, the term "subject" refers to any animal, including humans, monkeys, cows, horses, sheep, pigs, chickens, turkeys, quails, cats, dogs, mice, rats, rabbits, or guinea pigs that have developed or are susceptible to developing diseases caused or mediated by oxidative stress in vivo, particularly reactive oxygen species, and by administering the pharmaceutical composition of the present invention to the subject, the aforementioned diseases may be effectively prevented or treated. The pharmaceutical composition of the present invention may be administered in conjunction with existing therapeutic agents.

As used herein, the term "administration" refers to providing a predetermined substance to a patient by any suitable method, and the route of administration of the composition of the present invention may be any conventional route as long as it may reach the target tissue. It may be administered intraperitoneally, intravenously, intramuscularly, subcutaneously, intradermally, orally, topically, nasally, pulmonary, or rectally, but not limited thereto. In addition, the pharmaceutical composition of the present invention may be administered by any device capable of delivering the active substance to the target cells. Preferred modes of administration and formulations include intravenous injections, subcutaneous injections, intradermal injections, intramuscular injections, and drip infusions. Injections may be prepared using aqueous solvents such as physiological saline and Ringer's solution, non-aqueous solvents such as vegetable oils, higher fatty acid esters (e.g., ethyl oleate), and alcohols (e.g., ethanol, benzyl alcohol, propylene glycol, glycerin), and may include pharmaceutical carriers such as stabilizers to prevent deterioration (e.g., ascorbic acid, sodium bisulfite, sodium pyrosulfite, BHA, tocopherol, EDTA), emulsifiers, buffers for pH adjustment, and preservatives to inhibit microbial growth (e.g., phenylmercuric nitrate, thimerosal, benzalkonium chloride, phenol, cresol, benzyl alcohol).

In another aspect, the present invention provides a cosmetic composition including, as an active ingredient, one or more of the compounds represented by Chemical Formula 1, an isomer thereof, or a cosmetologically acceptable salt thereof. The cosmetic composition according to the present invention may provide antioxidant or antiinflammatory effects through topical application.

The content of the active ingredient in the cosmetic composition according to the present invention may be 0.00001 to 99.99 wt%, specifically 0.0001 to 50 wt%, and more specifically 0.002 to 30 wt%, based on the total weight of the composition, but not limited thereto.

As used herein, the term "antioxidant" refers to the action of inhibiting oxidation. The human body maintains a balance between pro-oxidants and antioxidants, but various factors may disrupt this balance, tilting it towards pro-oxidants, leading to oxidative stress, potential cell damage, and pathological diseases. Reactive oxygen species (ROS), which are the direct cause of this oxidative stress, are unstable and highly reactive, easily reacting with various biological substances and attacking macromolecules in the body, causing irreversible damage to cells and tissues, or leading to mutations, cytotoxicity, and carcinogenesis. Reactive nitrogen species (RNS) such as NO, HNO₂, and ONOO- are produced in large quantities during inflammatory responses due to the immune responses of macrophages, neutrophils, and other immune cells, along with ROS. These reactive oxygen species oxidize and destroy cells in the body, leading to exposure to various diseases. Therefore, by incorporating the compound of the present invention into cosmetics, antioxidant effects may be achieved, contributing to health promotion.

As used herein, the term "cosmetic composition" may be prepared in the form of general emulsion formulations and solubilized formulations. Examples of the emulsion formulations include nourishing lotion, cream, and essence, and examples of the solubilized formulations include softening lotion. Suitable formulations are not limited thereto, but may be, for example, in the form of solutions, gels, solid or paste anhydrous products, emulsions obtained by dispersing an oil phase in an aqueous phase, suspensions, microemulsions, microcapsules, microgranules, or ionic (liposomes) or nonionic vesicle dispersions, creams, skins, lotions, powders, ointments, sprays, or conceal sticks. It may also be in the form of foam or an aerosol composition containing a compressed propellant.

The cosmetic composition according to the present invention may additionally contain commonly used adjuvants such as fatty substances, organic solvents, solubilizers, thickeners and gelling agents, emollients, antioxidants, suspending agents, stabilizers, foaming agents, fragrances, surfactants, water, ionic or nonionic emulsifiers, fillers, sequestering agents, chelating agents, preservatives, vitamins, blockers, humectants, essential oils, dyes, pigments, hydrophilic or lipophilic active agents, lipid vesicles, or any other ingredients commonly used in cosmetic compositions.

Meanwhile, the compound of the present invention may exist in the form of a salt, particularly a pharmaceutically acceptable salt or a cosmetologically acceptable salt. As salts, salts commonly used in the art may be used without limitation, such as acid addition salts formed by pharmaceutically acceptable free acids. As used herein, the terms "pharmaceutically acceptable salt" and "cosmetologically acceptable salt" refer to any organic or inorganic addition salt of the compound represented by Chemical Formula 1 that is relatively non-toxic and harmless to the subject at a concentration that has an effective action and does not reduce the beneficial effects of the compound represented by Chemical Formula 1 due to side effects caused by the salt.

Acid addition salts may be prepared by conventional methods, for example, by dissolving the compound in an excess amount of aqueous acid solution and precipitating the salt using a water-miscible organic solvent such as methanol, ethanol, acetone, or acetonitrile. An equimolar amount of the compound and an acid or alcohol (e.g., glycol monomethyl ether) in water may be heated, and then the mixture may be evaporated to dryness, or the precipitated salt may be suction filtered.

In this case, organic acids and inorganic acids may be used as free acids, and inorganic acids such as hydrochloric acid, phosphoric acid, sulfuric acid, nitric acid, and tartaric acid may be used, and organic acids such as methanesulfonic acid, p-toluenesulfonic acid, acetic acid, trifluoroacetic acid, maleic acid, succinic acid, oxalic acid, benzoic acid, tartaric acid, fumaric acid, mandelic acid, propionic acid, citric acid, lactic acid, glycollic acid, gluconic acid, galacturonic acid, glutamic acid, glutaric acid, glucuronic acid, aspartic acid, ascorbic acid, carbonic acid, vanillic acid, and hydroiodic acid may be used, but not limited thereto.

In addition, pharmaceutically or cosmetologically acceptable metal salts may be prepared using bases. Alkali metal salts or alkaline earth metal salts may be obtained, for example, by dissolving the compound in an excess amount of alkali metal hydroxide or alkaline earth metal hydroxide solution, filtering off the insoluble compound salt, and then evaporating and drying the filtrate. In this case, it is pharmaceutically, cosmetically, or food-wise suitable to prepare sodium, potassium, or calcium salts in particular, but not limited thereto. In addition, the corresponding silver salts may be obtained by reacting the alkali metal or alkaline earth metal salts with a suitable silver salt (e.g., silver nitrate).

The pharmaceutically acceptable salts of the compounds of the present invention include, unless otherwise indicated, salts of acidic or basic groups that may be present in the compounds of Chemical Formula 1. For example, pharmaceutically or cosmetologically acceptable salts may include sodium, calcium, and potassium salts of hydroxy groups, and other pharmaceutically acceptable salts of amino groups may include hydrobromide, sulfate, hydrogen sulfate, phosphate, hydrogen phosphate, dihydrogen phosphate, acetate, succinate, citrate, tartrate, lactate, mandelate, methanesulfonate (mesylate), and p-toluenesulfonate (tosylate) salts, and may be prepared through methods known in the art.

In addition, the compound represented by Chemical Formula 1 according to the present invention includes, without limitation, not only its pharmaceutically acceptable salts but also possible solvates such as hydrates that may be prepared therefrom, and all possible stereoisomers. The solvates and stereoisomers of the compound represented by Chemical Formula 1 may be prepared from the compound represented by Chemical Formula 1 using methods known in the art.

In addition, the compound represented by Chemical Formula 1 according to the present invention may be prepared in crystalline or amorphous form, and if prepared in crystalline form, it may be arbitrarily hydrated or solvated. The present invention includes not only stoichiometric hydrates of the compound represented by Chemical Formula 1 but also compounds containing various amounts of water. The solvates of the compound represented by Chemical Formula 1 according to the present invention include both stoichiometric and non-stoichiometric solvates.

### ADVANTAGEOUS EFFECTS

The novel compound represented by Chemical Formula 1 according to the present invention may provide excellent antioxidant effects.

### BRIEF DESCRIPTION OF THE DRAWINGS

FIG. 1 is a diagram illustrating a reaction scheme for the preparation of 4-(((E)-3-(3,4-dihydroxyphenyl)acryloyl)oxy)benzyl(E)-3-(3,4-dihydroxyphenyl)acrylate according to an embodiment of the present invention.
FIG. 2 is a graph illustrating results of the cytotoxicity experiment according to Experimental Example 1.
FIG. 3 is a graph illustrating results of the antioxidant experiment according to Experimental Example 2.
FIG. 4 is a graph illustrating results of confirming the reactive oxygen species inhibitory ability according to Experimental Example 3.
FIG. 5 is images illustrating results of confirming the reactive oxygen species inhibitory ability using DCF-DA according to Experimental Example 3.

### MODE FOR CARRYING OUT THE INVENTION

Hereinafter, the present invention will be described in more detail through specific embodiments so that the person skilled in the art may easily implement it. However, the present invention may be implemented in various different forms and not limited to the embodiments described herein.

### Preparation Example 1: Preparation of (E)-3-(3,4-diacetoxyphenyl)acrylic acid

(*E*)-3-(3,4-dihydroxyphenyl)acrylic acid (3.0 g, 1.0 eq.) and pyridine (1.97 ml, 1.5 eq.) were placed in a reactor and cooled from room temperature to 0-5 °C. Acetic anhydride (8.5 g, 5.0 eq.) was slowly added for 10-20 minutes at the same temperature, and then the mixture was stirred at room temperature for 18 hours or more. Methylene chloride (10 ml) and n-heptane (30 ml) were added to the reaction mixture, and the mixture was stirred at room temperature for 1 hour or more. The solid compound was filtered and vacuum dried at 40 °C for 12 hours or more to obtain the compound (3.5 g, yield 79.7 %) according to the following Chemical Formula 2.

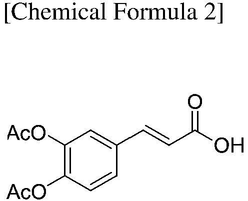

### Preparation Example 2: Preparation of 4-((E)-3-((4-(((E)-3-(3,4-diacetoxyphenyl)acryloyl)oxy)benzyl)oxy)-3-oxoprop-1-en-1-yl)-1,2-phenylene diacetate

(*E*)-3-(3,4-diacetoxyphenyl)acrylic acid (1.7 g, 2.1 eq.) prepared in Preparation Example 1 was placed in a reactor and cooled to 0-5 °C under a nitrogen atmosphere. Thionyl chloride (SOCl₂) (3.4 ml, excess (3-5 eq.)) was slowly added, and then the mixture was stirred at room temperature for 1 hour or more. Thionyl chloride was concentrated under reduced pressure, and then anhydrous methylene chloride (10 ml) was added under a nitrogen atmosphere (reactor A). In reactor B, gastrodigenin (Aldrich) (0.4 g, 1.0 eq.), anhydrous methylene chloride (5 ml), and triethylamine (0.7 g, 2.2 eq.) were added and stirred at room temperature for 30 minutes or more. The material in reactor A was slowly added to reactor B at room temperature for 10-20 minutes, and then the mixture was refluxed with stirring for 2 hours or more. After completion of the reaction, the reaction mixture was concentrated under reduced pressure, and then separated by column chromatography using ethyl acetate/n-heptane as the developing solvent to obtain the compound (1.1 g, yield 55.5 %) according to the following Chemical Formula 3.

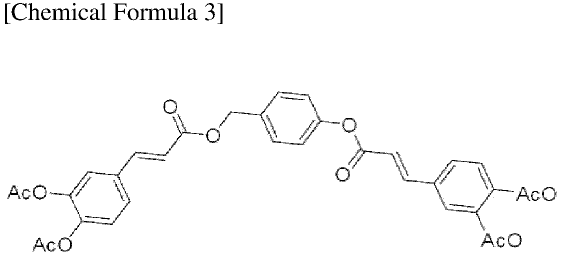

¹H-NMR (400MHz, DMSO-*d6*) *δ* 7.84-7.68 (m, 6H), 7.52-7.50 (d, 2H), 7.38-7.31 (m, 2H), 7.26-7.23 (d, 2H), 6.92-6.88 (d, 1H), 6.75-6.71 (d, 1H), 5.25 (s, 2H), 2.31-2.28 (m, 12H).

### Embodiment 1: Preparation of 4-(((E)-3-(3,4-dihydroxyphenyl)acryloyl)oxy)benzyl (E)-3-(3,4-dihydroxyphenyl)acrylate

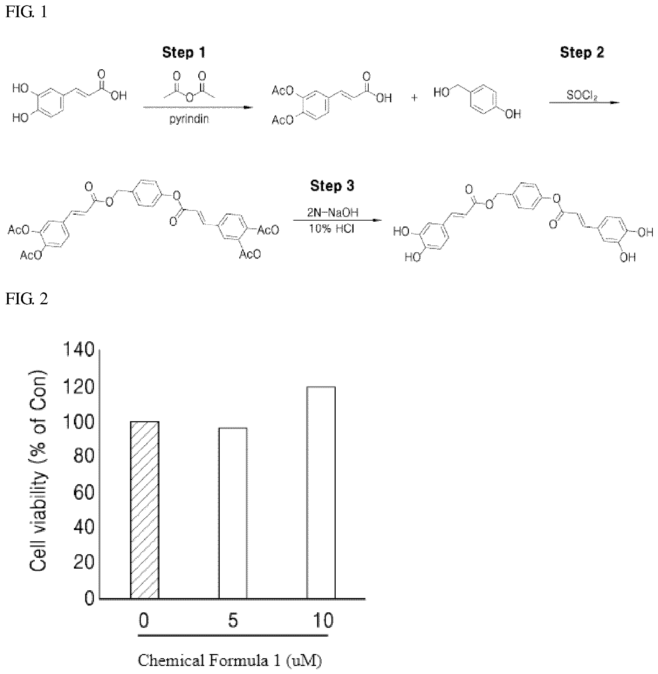

FIG. 1 is a diagram illustrating the reaction scheme for the preparation of 4-(((*E*)-3-(3,4-dihydroxyphenyl)acryloyl)oxy)benzyl (*E*)-3-(3,4-dihydroxyphenyl)acrylate.

4-((*E*)-3-((4-(((*E*)-3-(3,4-diacetoxyphenyl)acryloyl)oxy)benzyl)oxy)-3-oxoprop-1-en-1-yl)-1,2-phenylene diacetate (0.5 g, 1 eq.) prepared in Preparation Example 2 and dioxane (5 ml) were placed in a reactor, and 2N sodium hydroxide (NaOH) (2.0 ml, 5 eq.) was added, followed by stirring at 50 °C for 3 hours or more. After completion of the reaction, the mixture was cooled to room temperature, and 10 % hydrochloric acid (HCl) (10 ml) and ethyl acetate (15 ml) were added, followed by stirring for 30 minutes or more. The organic layer was extracted, dehydrated with magnesium sulfate (MgSO₄), and concentrated under reduced pressure. Vacuum drying at 40 °C for 10 hours or more to obtain the compound (0.18 g, yield 50.0 %) according to Chemical Formula 4.

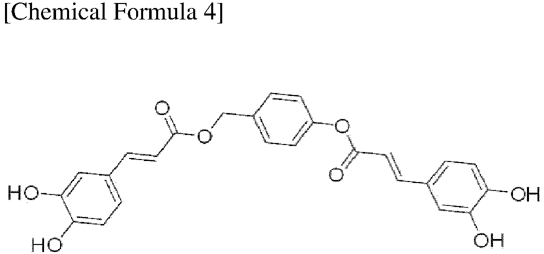

¹H-NMR (400MHz, DMSO-*d6*) *δ* 7.70-7.66 (d, 1H), 7.55-7.51 (d, 1H), 7.49-7.47 (d, 2H), 7.21-7.19 (d, 2H), 7.14-7.01 (m, 4H), 6.80-6.75 (m, 2H), 6.51-6.47 (d, 1H), 6.35-6.31 (d, 1H),

MS (ESI+): m/z 449.12 [M+H]⁺.

### Embodiment 2: Preparation of (E)-4-(((E)-3-(4-acetoxy-3-hydroxyphenyl)acryloyl)oxy)benzyl-3-(4-acetoxy-3-hydroxyphenyl)acrylate

(E)-3-(3-hydroxy, 4-acetoxyphenyl)acrylic acid (purchased from Sigma-Aldrich) was placed in a reactor and cooled to 0-5 °C under a nitrogen atmosphere. Thionyl chloride (SOCl₂) (3.4 ml, excess (3-5 eq.)) was slowly added, and then the mixture was stirred at room temperature for 1 hour or more. Thionyl chloride was concentrated under reduced pressure, and then anhydrous methylene chloride (10 ml) was added under a nitrogen atmosphere (reactor A). In reactor B, gastrodigenin (0.4 g, 1.0 eq.), anhydrous methylene chloride (5 ml), and triethylamine (0.7 g, 2.2 eq.) were added and stirred at room temperature for 30 minutes or more. The material in reactor A was slowly added to reactor B at room temperature for 10-20 minutes, and then the mixture was refluxed with stirring for 2 hours or more. After completion of the reaction, the reaction mixture was concentrated under reduced pressure, and then separated by column chromatography using ethyl acetate/n-heptane as the developing solvent to obtain the compound according to the following Chemical Formula 5.

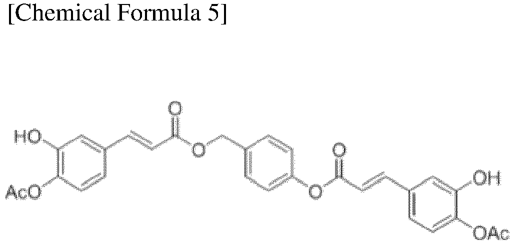

¹H-NMR (400MHz, DMSO-*d6*) *δ* 9.46 (s, 2H), 7.55-7.53 (d, 2H), 7.25-7.19 (m, 6H), 6.80-6.75 (m, 4H), 6.35-6.31 (d, 2H), 5.2 (s, 2H), 2.18 (s, 6H)

MS(ESI+): m/z 533.12 [M+H]⁺.

### Embodiment 3: Preparation of (E)-4-(((E)-3-(4-acetoxy-3-methoxyphenyl)acryloyl)oxy)benzyl-3-(4-acetoxy-3-methoxyphenyl)acrylate

(E)-3-(3-Methoxy, 4-acetoxyphenyl)acrylic acid (purchased from Sigma-Aldrich) was placed in a reactor and cooled to 0-5 °C under a nitrogen atmosphere. Thionyl chloride (SOCl₂) (3.4 ml, excess (3-5 eq.)) was slowly added, and then the mixture was stirred at room temperature for 1 hour or more. Thionyl chloride was concentrated under reduced pressure, and then anhydrous methylene chloride (10 ml) was added under a nitrogen atmosphere (reactor A). In reactor B, gastrodigenin (0.4 g, 1.0 eq.), anhydrous methylene chloride (5 ml), and triethylamine (0.7 g, 2.2 eq.) were added and stirred at room temperature for 30 minutes or more. The material in reactor A was slowly added to reactor B at room temperature for 10-20 minutes, and then the mixture was refluxed with stirring for 2 hours or more. After completion of the reaction, the reaction mixture was concentrated under reduced pressure, and then separated by column chromatography using ethyl acetate/n-heptane as the developing solvent to obtain the compound according to the following Chemical Formula 6.

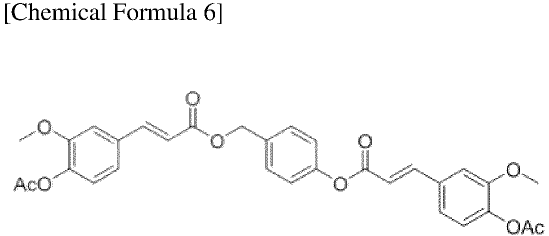

¹H-NMR (400MHz, DMSO-*d6*) *δ* 7.55-7.53 (d, 2H), 7.25-7.19 (m, 6H), 6.80-6.75 (m, 4H), 6.35-6.31 (d, 2H), 5.2 (s, 2H), 3.68 (s, 6H), 2.18 (s, 6H)

MS (ESI+): m/z 561.12 [M+H]⁺.

### Embodiment 4: Preparation of (E)-4-(((E)-3-(4-acetoxy-3-hydroxyphenyl)acryloyl)oxy)-2-methylbenzyl-3-(4-acetoxy-3-hydroxyphenyl)acrylate

(E)-3-(3-hydroxy, 4-acetoxyphenyl)acrylic acid (purchased from Sigma-Aldrich) was placed in a reactor and cooled to 0-5 °C under a nitrogen atmosphere. Thionyl chloride (SOCl₂) (3.4 ml, excess (3-5 eq.)) was slowly added, and then the mixture was stirred at room temperature for 1 hour or more. Thionyl chloride was concentrated under reduced pressure, and then anhydrous methylene chloride (10 ml) was added under a nitrogen atmosphere (reactor A). In reactor B, 2-methyl, 4-hydroxybenzyl alcohol (0.4 g, 1.0 eq.), anhydrous methylene chloride (5 ml), and triethylamine (0.7 g, 2.2 eq.) were added and stirred at room temperature for 30 minutes or more. The material in reactor A was slowly added to reactor B at room temperature for 10-20 minutes, and then the mixture was refluxed with stirring for 2 hours or more. After completion of the reaction, the reaction mixture was concentrated under reduced pressure, and then separated by column chromatography using ethyl acetate/n-heptane as the developing solvent to obtain the compound according to the following Chemical Formula 7.

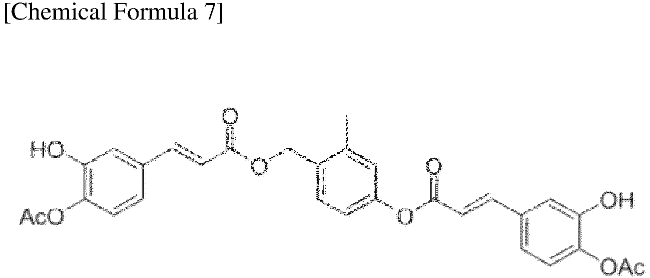

¹H-NMR (400MHz, DMSO-*d6*) *δ* 9.46 (s, 2H), 7.55-7.53 (d, 2H), 7.25-7.19 (m, 5H), 6.80-6.75 (m, 4H), 6.35-6.31 (d, 2H), 5.2 (s, 2H), 2.31 (s, 3H), 2.18 (s, 6H)

MS (ESI+): m/z 547.15 [M+H]⁺.

### Embodiment 5: Preparation of (E)-4-(((E)-3-(4-acetoxv-3-methoxyphenyl)acryloyl)oxy)-2-methylbenzyl-3-(4-acetoxy-3-methoxyphenyl)acrylate

(E)-3-(3-Methoxy, 4-acetoxyphenyl)acrylic acid (purchased from Sigma-Aldrich) was placed in a reactor and cooled to 0-5 °C under a nitrogen atmosphere. Thionyl chloride (SOCl₂) (3.4 ml, excess (3-5 eq.)) was slowly added, and then the mixture was stirred at room temperature for 1 hour or more. Thionyl chloride was concentrated under reduced pressure, and then anhydrous methylene chloride (10 ml) was added under a nitrogen atmosphere (reactor A). In reactor B, 2-methyl, 4-hydroxybenzyl alcohol (0.4 g, 1.0 eq.), anhydrous methylene chloride (5 ml), and triethylamine (0.7 g, 2.2 eq.) were added and stirred at room temperature for 30 minutes or more. The material in reactor A was slowly added to reactor B at room temperature for 10-20 minutes, and then the mixture was refluxed with stirring for 2 hours or more. After completion of the reaction, the reaction mixture was concentrated under reduced pressure, and then separated by column chromatography using ethyl acetate/n-heptane as the developing solvent to obtain the compound according to the following Chemical Formula 8.

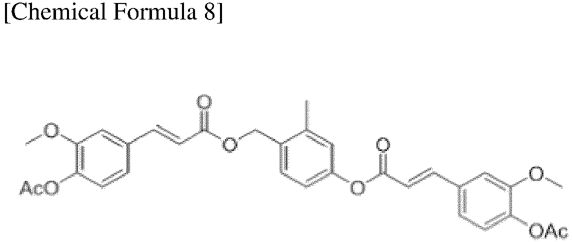

¹H-NMR (400MHz, DMSO-*d6*) *δ* 7.55-7.53 (d, 2H), 7.25-7.19 (m, 5H), 6.80-6.75 (m, 4H), 6.35-6.31 (d, 2H), 5.2 (s, 2H), 3.68 (s, 6H), 2.29 (s, 3H), 2.18 (s, 6H).

MS (ESI+): m/z 575.16 [M+H]⁺.

### Embodiment 6: Preparation of (E)-4-(((E)-3-(4-acetoxy-3-hydroxyphenyl)acryloyl)oxy)-3-methylbenzyl-3-(4-acetoxy-3-hydroxyphenyl)acrylate

(E)-3-(3-hydroxy, 4-acetoxyphenyl)acrylic acid (purchased from Sigma-Aldrich) was placed in a reactor and cooled to 0-5 °C under a nitrogen atmosphere. Thionyl chloride (SOCl₂) (3.4 ml, excess (3-5 eq.)) was slowly added, and then the mixture was stirred at room temperature for 1 hour or more. Thionyl chloride was concentrated under reduced pressure, and then anhydrous methylene chloride (10 ml) was added under a nitrogen atmosphere (reactor A). In reactor B, 3-methyl, 4-hydroxybenzyl alcohol (0.4 g, 1.0 eq.), anhydrous methylene chloride (5 ml), and triethylamine (0.7 g, 2.2 eq.) were added and stirred at room temperature for 30 minutes or more. The material in reactor A was slowly added to reactor B at room temperature for 10-20 minutes, and then the mixture was refluxed with stirring for 2 hours or more. After completion of the reaction, the reaction mixture was concentrated under reduced pressure, and then separated by column chromatography using ethyl acetate/n-heptane as the developing solvent to obtain the compound according to the following Chemical Formula 9.

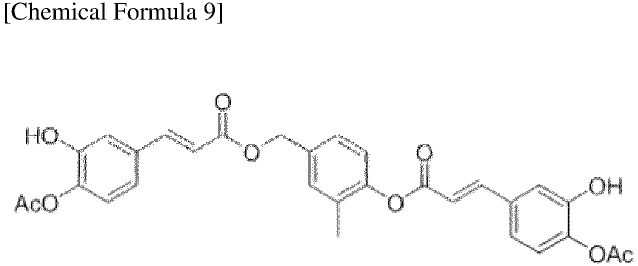

¹H-NMR (400MHz, DMSO-*d6*) *δ* 9.46 (s, 2H), 7.55-7.53 (d, 2H), 7.25-7.19 (m, 5H), 6.80-6.75 (m, 4H), 6.35-6.31 (d, 2H), 5.2 (s, 2H), 2.18 (s, 6H), 2.15 (s, 3H).

MS (ESI+): m/z 547.15 [M+H]⁺.

### Embodiment 7: Preparation of (E)-4-(((E)-3-(4-acetoxy-3-methoxyphenyl)acryloyl)oxy)-3-methylbenzyl-3-(4-acetoxy-3-methoxyphenyl)acrylate

(E)-3-(3-methoxy, 4-acetoxyphenyl)acrylic acid (purchased from Sigma-Aldrich) was placed in a reactor and cooled to 0-5 °C under a nitrogen atmosphere. Thionyl chloride (SOCl₂) (3.4 ml, excess (3-5 eq.)) was slowly added, and then the mixture was stirred at room temperature for 1 hour or more. Thionyl chloride was concentrated under reduced pressure, and then anhydrous methylene chloride (10 ml) was added under a nitrogen atmosphere (reactor A). In reactor B, 3-methyl, 4-hydroxybenzyl alcohol (0.4 g, 1.0 eq.), anhydrous methylene chloride (5 ml), and triethylamine (0.7 g, 2.2 eq.) were added and stirred at room temperature for 30 minutes or more. The material in reactor A was slowly added to reactor B at room temperature for 10-20 minutes, and then the mixture was refluxed with stirring for 2 hours or more. After completion of the reaction, the reaction mixture was concentrated under reduced pressure, and then separated by column chromatography using ethyl acetate/n-heptane as the developing solvent to obtain the compound according to the following Chemical Formula 10.

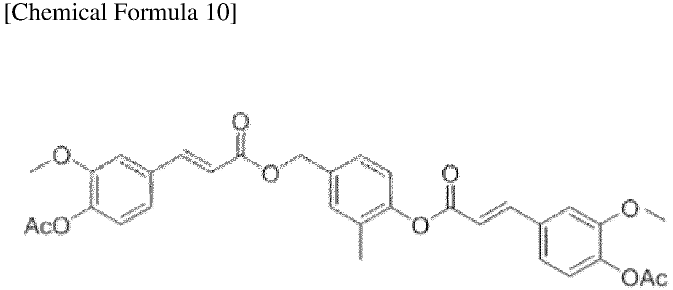

¹H-NMR (400MHz, DMSO-*d6*) *δ* 9.46 (s, 2H), 7.55-7.53 (d, 2H), 7.25-7.19 (m, 5H), 6.80-6.75 (m, 4H), 6.35-6.31 (d, 2H), 5.2 (s, 2H), 2.18 (s, 6H), 2.15 (s, 3H).

MS (ESI+): m/z 547.15 [M+H]⁺.

### Embodiment 8: Preparation of (E)-4-(((E)-3-(4-acetoxy-3-hydroxyphenyl)acryloyl)oxy)-2-methoxybenzyl-3-(4-acetoxy-3-hydroxyphenyl)acrylate

(E)-3-(3-hydroxy, 4-acetoxyphenyl)acrylic acid (purchased from Sigma-Aldrich) was placed in a reactor and cooled to 0-5 °C under a nitrogen atmosphere. Thionyl chloride (SOCl₂) (3.4 ml, excess (3-5 eq.)) was slowly added, and then the mixture was stirred at room temperature for 1 hour or more. Thionyl chloride was concentrated under reduced pressure, and then anhydrous methylene chloride (10 ml) was added under a nitrogen atmosphere (reactor A). In reactor B, 2-methoxy, 4-hydroxybenzyl alcohol (0.4 g, 1.0 eq.), anhydrous methylene chloride (5 ml), and triethylamine (0.7 g, 2.2 eq.) were added and stirred at room temperature for 30 minutes or more. The material in reactor A was slowly added to reactor B at room temperature for 10-20 minutes, and then the mixture was refluxed with stirring for 2 hours or more. After completion of the reaction, the reaction mixture was concentrated under reduced pressure, and then separated by column chromatography using ethyl acetate/n-heptane as the developing solvent to obtain the compound according to the following Chemical Formula 11.

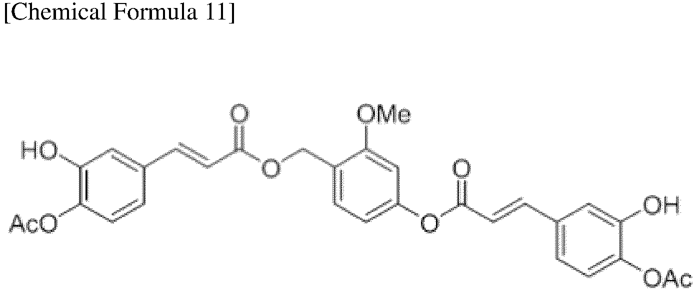

¹H-NMR (400MHz, DMSO-*d6*) *δ* 9.46 (s, 2H), 7.55-7.53 (d, 2H), 7.25-7.19 (m, 5H), 6.80-6.75 (m, 4H), 6.35-6.31 (d, 2H), 5.2 (s, 2H), 3.73 (s, 3H), 2.18 (s, 6H)

MS (ESI+): m/z 563.15 [M+H]⁺.

### Embodiment 9: Preparation of (E)-4-(((E)-3-(4-acetoxy-3-methoxyphenyl)acryloyl)oxy)-2-methoxybenzyl-3-(4-acetoxy-3-methoxyphenyl)acrylate

(E)-3-(3-Methoxy, 4-acetoxyphenyl)acrylic acid (purchased from Sigma-Aldrich) was placed in a reactor and cooled to 0-5 °C under a nitrogen atmosphere. Thionyl chloride (SOCl₂) (3.4 ml, excess (3-5 eq.)) was slowly added, and then the mixture was stirred at room temperature for 1 hour or more. Thionyl chloride was concentrated under reduced pressure, and then anhydrous methylene chloride (10 ml) was added under a nitrogen atmosphere (reactor A). In reactor B, 2-methoxy, 4-hydroxybenzyl alcohol (0.4 g, 1.0 eq.), anhydrous methylene chloride (5 ml), and triethylamine (0.7 g, 2.2 eq.) were added and stirred at room temperature for 30 minutes or more. The material in reactor A was slowly added to reactor B at room temperature for 10-20 minutes, and then the mixture was refluxed with stirring for 2 hours or more. After completion of the reaction, the reaction mixture was concentrated under reduced pressure, and then separated by column chromatography using ethyl acetate/n-heptane as the developing solvent to obtain the compound according to the following Chemical Formula 12.

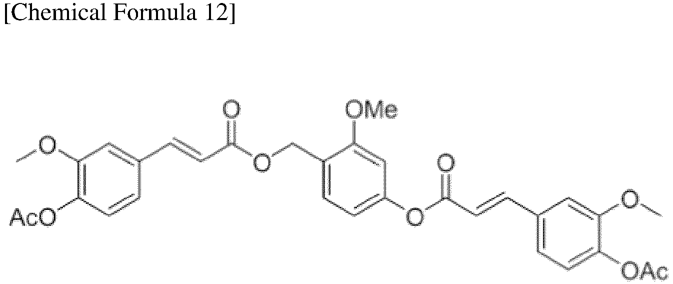

¹H-NMR (400MHz, DMSO-d6) *δ* 7.55-7.53 (d, 2H), 7.25-7.19 (m, 5H), 6.80-6.75 (m, 4H), 6.35-6.31 (d, 2H), 5.2 (s, 2H), 3.73 (s, 3H), 3.68 (s, 6H), 2.18 (s, 6H)

MS (ESI+): m/z 591.15 [M+H]⁺.

### Embodiment 10: Preparation of (E)-4-(((E)-3-(4-hydroxy-3-methoxyphenyl)acryloyl)oxy)-2-methylbenzyl-3-(4-hydroxy-3-methoxyphenyl)acrylate

(E)-3-(3-methoxy, 4-hydroxyphenyl)acrylic acid (purchased from Sigma-Aldrich) was placed in a reactor and cooled to 0-5 °C under a nitrogen atmosphere. Thionyl chloride (SOCl₂) (3.4 ml, excess (3-5 eq.)) was slowly added, and then the mixture was stirred at room temperature for 1 hour or more. Thionyl chloride was concentrated under reduced pressure, and then anhydrous methylene chloride (10 ml) was added under a nitrogen atmosphere (reactor A). In reactor B, 2-methyl, 4-hydroxybenzyl alcohol (0.4 g, 1.0 eq.), anhydrous methylene chloride (5 ml), and triethylamine (0.7 g, 2.2 eq.) were added and stirred at room temperature for 30 minutes or more. The material in reactor A was slowly added to reactor B at room temperature for 10-20 minutes, and then the mixture was refluxed with stirring for 2 hours or more. After completion of the reaction, the reaction mixture was concentrated under reduced pressure, and then separated by column chromatography using ethyl acetate/n-heptane as the developing solvent to obtain the compound according to the following Chemical Formula 13.

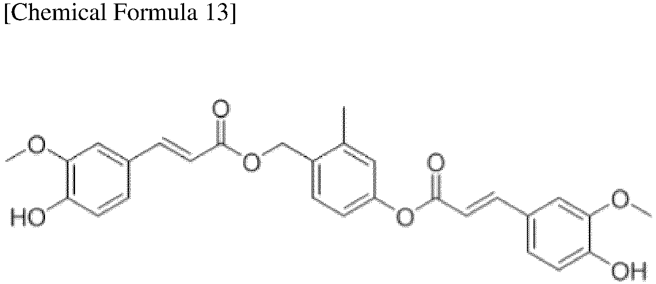

¹H-NMR (400MHz, DMSO-*d6*) *δ* 9.55 (s, 2H), 7.55-7.53 (d, 2H), 7.25-7.19 (m, 5H), 6.80-6.75 (m, 4H), 6.35-6.31 (d, 2H), 5.2 (s, 2H), 3.68 (s, 6H), 2.29 (s, 3H).

MS (ESI+): m/z 491.16 [M+H]⁺.

### Embodiment 11: Preparation of (E)-4-(((E)-3-(4-hydroxy-3-methoxyphenyl)acryloyl)oxy)-3-methylbenzyl-3-(4-hydroxy-3-methoxyphenyl)acrylate

(E)-3-(3-methoxy, 4-hydroxyphenyl)acrylic acid (purchased from Sigma-Aldrich) was placed in a reactor and cooled to 0-5 °C under a nitrogen atmosphere. Thionyl chloride (SOCl₂) (3.4 ml, excess (3-5 eq.)) was slowly added, and then the mixture was stirred at room temperature for 1 hour or more. Thionyl chloride was concentrated under reduced pressure, and then anhydrous methylene chloride (10 ml) was added under a nitrogen atmosphere (reactor A). In reactor B, 3-methyl, 4-hydroxybenzyl alcohol (0.4 g, 1.0 eq.), anhydrous methylene chloride (5 ml), and triethylamine (0.7 g, 2.2 eq.) were added and stirred at room temperature for 30 minutes or more. The material in reactor A was slowly added to reactor B at room temperature for 10-20 minutes, and then the mixture was refluxed with stirring for 2 hours or more. After completion of the reaction, the reaction mixture was concentrated under reduced pressure, and then separated by column chromatography using ethyl acetate/n-heptane as the developing solvent to obtain the compound according to the following Chemical Formula 14.

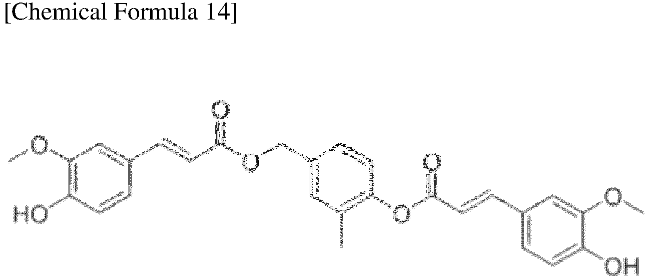

¹H-NMR (400MHz, DMSO-d6) *δ* 9.55 (s, 2H), 7.55-7.53 (d, 2H), 7.25-7.19 (m, 5H), 6.80-6.75 (m, 4H), 6.35-6.31 (d, 2H), 5.2 (s, 2H), 3.68 (s, 6H), 2.15 (s, 3H).

MS (ESI+): m/z 491.16 [M+H]⁺.

### Embodiment 12: Preparation of (E)-4-(((E)-3-(3,4-dimethoxyphenyl)acryloyl)oxy)-2-methoxybenzyl-3-(3,4-dimethoxyphenyl)acrylate

(E)-3-(3,4-dimethoxyphenyl)acrylic acid (purchased from Sigma-Aldrich) was placed in a reactor and cooled to 0-5 °C under a nitrogen atmosphere. Thionyl chloride (SOCl₂) (3.4 ml, excess (3-5 eq.)) was slowly added, and then the mixture was stirred at room temperature for 1 hour or more. Thionyl chloride was concentrated under reduced pressure, and then anhydrous methylene chloride (10 ml) was added under a nitrogen atmosphere (reactor A). In reactor B, 2-methyl, 4-hydroxybenzyl alcohol (0.4 g, 1.0 eq.), anhydrous methylene chloride (5 ml), and triethylamine (0.7 g, 2.2 eq.) were added and stirred at room temperature for 30 minutes or more. The material in reactor A was slowly added to reactor B at room temperature for 10-20 minutes, and then the mixture was refluxed with stirring for 2 hours or more. After completion of the reaction, the reaction mixture was concentrated under reduced pressure, and then separated by column chromatography using ethyl acetate/n-heptane as the developing solvent to obtain the compound according to the following Chemical Formula 15.

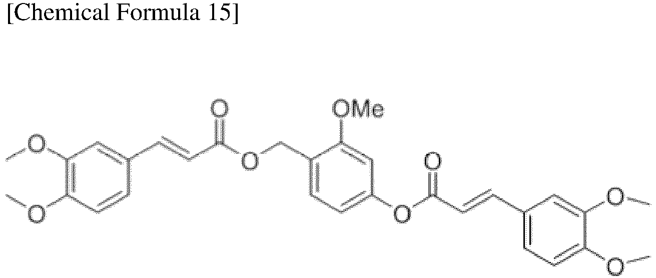

¹H-NMR (400MHz, DMSO-d6) *δ* 7.55-7.53 (d, 2H), 7.25-7.19 (m, 5H), 6.80-6.75 (m, 4H), 6.35-6.31 (d, 2H), 5.2 (s, 2H), 3.73 (s, 3H), 3.68 (s, 12H).

MS (ESI+): m/z 535.15 [M+H]⁺.

### Embodiment 13: Preparation of (E)-4-(((E)-3-(3,4-dihydroxyphenyl)acryloyl)oxy)-3-fluorobenzyl-3-(3,4-dihydroxyphenyl)acrylate

(E)-3-(3,4-diacetoxyphenyl)acrylic acid prepared in Preparation Example 1 was placed in a reactor and cooled to 0-5 °C under a nitrogen atmosphere. Thionyl chloride (SOCl₂) (3.4 ml, excess (3-5 eq.)) was slowly added, and then the mixture was stirred at room temperature for 1 hour or more. Thionyl chloride was concentrated under reduced pressure, and then anhydrous methylene chloride (10 ml) was added under a nitrogen atmosphere (reactor A). In reactor B, 3-fluoro, 4-hydroxybenzyl alcohol (0.4 g, 1.0 eq.), anhydrous methylene chloride (5 ml), and triethylamine (0.7 g, 2.2 eq.) were added and stirred at room temperature for 30 minutes or more. The material in reactor A was slowly added to reactor B at room temperature for 10-20 minutes, and then the mixture was refluxed with stirring for 2 hours or more. After completion of the reaction, the reaction mixture was concentrated under reduced pressure, and then separated by column chromatography using ethyl acetate/n-heptane as the developing solvent to obtain the compound according to the following Chemical Formula 16.

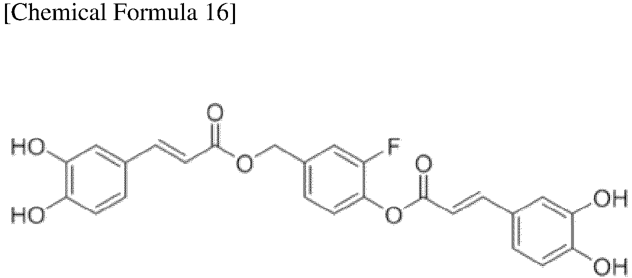

¹H-NMR (400MHz, DMSO-*d*6) *δ* 9.55 (s, 4H), 7.55-7.53 (d, 2H), 7.25-7.19 (m, 5H), 6.80-6.75 (m, 4H), 6.35-6.31 (d, 2H), 5.2 (s, 2H).

MS (ESI+): m/z 467.11 [M+H]⁺.

### Embodiment 14: Preparation of (E)-4-(((E)-3-(4-acetoxy-3-hydroxyphenyl)acryloyl)oxy)-3-chlorobenzyl-3-(4-acetoxy-3-hydroxyphenyl)acrylate

(E)-3-(3-hydroxy, 4-acetoxyphenyl)acrylic acid (purchased from Sigma-Aldrich) was placed in a reactor and cooled to 0-5 °C under a nitrogen atmosphere. Thionyl chloride (SOCl₂) (3.4 ml, excess (3-5 eq.)) was slowly added, and then the mixture was stirred at room temperature for 1 hour or more. Thionyl chloride was concentrated under reduced pressure, and then anhydrous methylene chloride (10 ml) was added under a nitrogen atmosphere (reactor A). In reactor B, 3-chloro, 4-hydroxybenzyl alcohol (0.4 g, 1.0 eq.), anhydrous methylene chloride (5 ml), and triethylamine (0.7 g, 2.2 eq.) were added and stirred at room temperature for 30 minutes or more. The material in reactor A was slowly added to reactor B at room temperature for 10-20 minutes, and then the mixture was refluxed with stirring for 2 hours or more. After completion of the reaction, the reaction mixture was concentrated under reduced pressure, and then separated by column chromatography using ethyl acetate/n-heptane as the developing solvent to obtain the compound according to the following Chemical Formula 17.

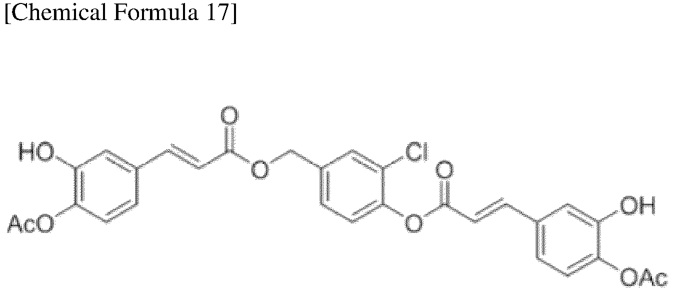

¹H-NMR (400MHz, DMSO-*d6*) *δ* 9.55 (s, 2H), 7.55-7.53 (d, 2H), 7.25-7.19 (m, 5H), 6.80-6.75 (m, 4H), 6.35-6.31 (d, 2H), 5.2 (s, 2H), 2.18 (s, 6H).

MS (ESI+): m/z 567.11 [M+H]⁺.

### Embodiment 15: Preparation of (E)-3-fluoro-4-((((E)-3-(4-hydroxy-3-methoxyphenyl)acryloyl)oxy)methyl)phenyl-3-(4-hydroxy-3-methoxyphenyl)acrylate

(E)-3-(3-Methoxy, 4-hydroxyphenyl)acrylic acid (purchased from Sigma-Aldrich) was placed in a reactor and cooled to 0-5 °C under a nitrogen atmosphere. Thionyl chloride (SOCl₂) (3.4 ml, excess (3-5 eq.)) was slowly added, and then the mixture was stirred at room temperature for 1 hour or more. Thionyl chloride was concentrated under reduced pressure, and then anhydrous methylene chloride (10 ml) was added under a nitrogen atmosphere (reactor A). In reactor B, 2-fluoro, 4-hydroxybenzyl alcohol (0.4 g, 1.0 eq.), anhydrous methylene chloride (5 ml), and triethylamine (0.7 g, 2.2 eq.) were added and stirred at room temperature for 30 minutes or more. The material in reactor A was slowly added to reactor B at room temperature for 10-20 minutes, and then the mixture was refluxed with stirring for 2 hours or more. After completion of the reaction, the reaction mixture was concentrated under reduced pressure, and then separated by column chromatography using ethyl acetate/n-heptane as the developing solvent to obtain the compound according to the following Chemical Formula 18.

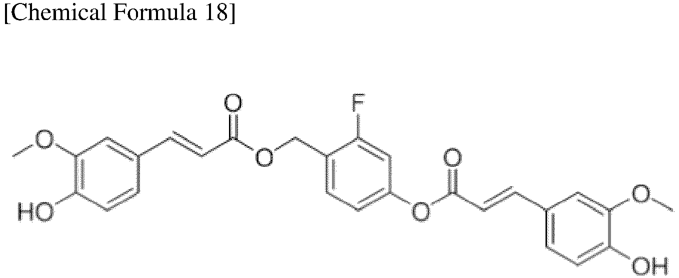

¹H-NMR (400MHz, DMSO-*d6*) *δ* 9.55 (s, 2H), 7.55-7.53 (d, 2H), 7.25-7.19 (m, 5H), 6.80-6.75 (m, 4H), 6.35-6.31 (d, 2H), 5.2 (s, 2H), 3.68 (s, 6H).

MS (ESI+): m/z 495.11 [M+H]⁺.

### Embodiment 16: Preparation of (E)-4-(((E)-3-(4-acetoxy-3-methoxyphenyl)acryloyl)oxy)-2-fluorobenzyl-3-(4-acetoxy-3-methoxyphenyl)acrylate

(E)-3-(3-Methoxy, 4-acetoxyphenyl)acrylic acid (purchased from Sigma-Aldrich) was placed in a reactor and cooled to 0-5 °C under a nitrogen atmosphere. Thionyl chloride (SOCl₂) (3.4 ml, excess (3-5 eq.)) was slowly added, and then the mixture was stirred at room temperature for 1 hour or more. Thionyl chloride was concentrated under reduced pressure, and then anhydrous methylene chloride (10 ml) was added under a nitrogen atmosphere (reactor A). In reactor B, 2-fluoro, 4-hydroxybenzyl alcohol (0.4 g, 1.0 eq.), anhydrous methylene chloride (5 ml), and triethylamine (0.7 g, 2.2 eq.) were added and stirred at room temperature for 30 minutes or more. The material in reactor A was slowly added to reactor B at room temperature for 10-20 minutes, and then the mixture was refluxed with stirring for 2 hours or more. After completion of the reaction, the reaction mixture was concentrated under reduced pressure, and then separated by column chromatography using ethyl acetate/n-heptane as the developing solvent to obtain the compound according to the following Chemical Formula 19.

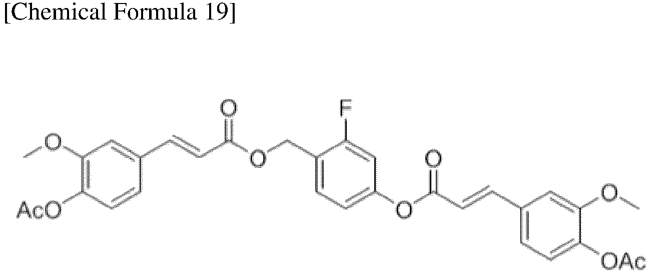

¹H-NMR (400MHz, DMSO-*d6*) *δ* 7.55-7.53 (d, 2H), 7.35-7.19 (m, 5H), 6.80-6.75 (m, 4H), 6.35-6.31 (d, 2H), 5.21 (s, 2H), 3.68 (s, 6H), 2.18 (s, 6H).

MS (ESI+): m/z 579.16 [M+H]⁺.

### Embodiment 17: Preparation of (E)-4-(((E)-3-(4-acetoxy-3-methoxyphenyl)acryloyl)oxy)-3-fluorobenzyl-3-(4-acetoxy-3-methoxyphenyl)acrylate

(E)-3-(3-Methoxy 4-acetoxyphenyl)acrylic acid (purchased from Sigma-Aldrich) was placed in a reactor and cooled to 0-5 °C under a nitrogen atmosphere. Thionyl chloride (SOCl₂) (3.4ml, excess (3-5 eq.)) was slowly added, and then the mixture was stirred at room temperature for 1 hour or more. Thionyl chloride was concentrated under reduced pressure, and then anhydrous methylene chloride (10 ml) was added under a nitrogen atmosphere (reactor A). In reactor B, 3-Fluoro,4-hydroxybenzyl alcohol (0.4 g, 1.0 eq.), anhydrous methylene chloride (5 ml), and triethylamine (0.7 g, 2.2 eq.) were added and stirred at room temperature for 30 minutes or more. The material in reactor A was slowly added to reactor B for 10-20 minutes at room temperature, and the mixture was refluxed and stirred for 2 hours or more. After completion of the reaction, the reaction mixture was concentrated under reduced pressure, and then separated by column chromatography using ethyl acetate/n-heptane as an eluent to obtain the compound according to the following Chemical Formula 20.

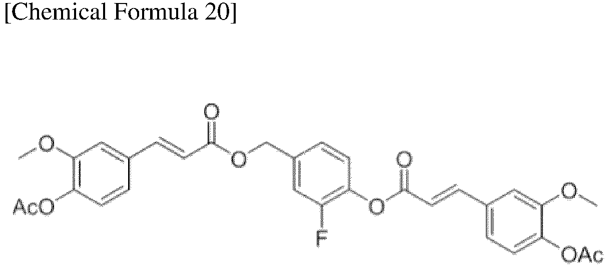

¹H-NMR (400MHz, DMSO-*d6*) *δ* 7.55-7.53 (d, 2H), 7.35-7.19 (m, 5H), 6.94-6.73 (m, 4H), 6.35-6.31 (d, 2H), 5.12 (s, 2H), 3.68 (s, 6H), 2.18 (s, 6H).

MS (ESI+): m/z 579.16 [M+H]⁺.

### Embodiment 18: Preparation of (E)-2-fluoro-4-((((E)-3-(4-hydroxy-3-methoxyphenyl)acryloyl)oxy)methyl)phenyl-3-(4-hydroxy-3-methoxyphenyl)acrylate

(E)-4-(((E)-3-(4-acetoxy-3-methoxyphenyl)acryloyl)oxy)-3-fluorobenzyl-3-(4-acetoxy-3-methoxyphenyl)acrylate (0.5 g, 1 eq.), obtained from Embodiment 17 and dioxane (5 ml) were placed in a reactor, and 2N sodium hydroxide (NaOH) (2.0 ml, 5 eq.) was added, followed by stirring at 50 °C for 3 hours or more. After completion of the reaction, the mixture was cooled to room temperature, and 10 % hydrochloric acid (HCl) (10 ml) and ethyl acetate (15 ml) were added, followed by stirring for 30 minutes or more. The organic layer was extracted, dehydrated with magnesium sulfate (MgSO₄), and concentrated under reduced pressure. Vacuum drying at 40 °C for 10 hours or more to obtain the compound according to Chemical Formula 21.

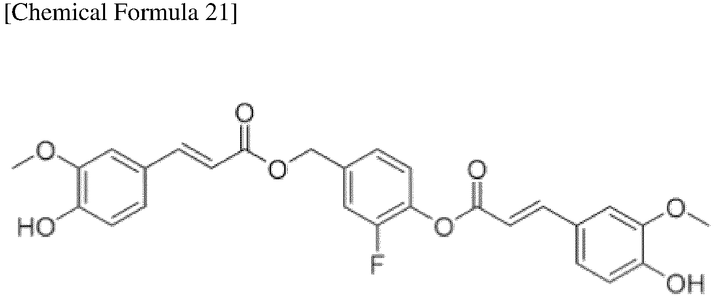

¹H-NMR (400MHz, DMSO-*d6*) *δ* 9.55 (s, 2H), 7.55-7.53 (d, 2H), 7.35-7.19 (m, 5H), 6.94-6.73 (m, 4H), 6.35-6.31 (d, 2H), 5.12 (s, 2H), 3.68 (s, 6H).

MS (ESI+): m/z 495.16 [M+H]⁺.

### Experimental Example 1

To confirm whether the compound prepared in Embodiment 1 causes toxicity in skin cells and induces skin irritation, a WST-1 assay was conducted, and the results are shown in Table 1 and FIG. 2 below. In the experiment, human keratinocytes (HaCaT cells) were seeded at a density of 1×10⁴ cells per well in a 96-well plate with DMEM (Dulbecco's Modified Eagle Medium) containing 10 % FBS (Fetal Bovine Serum) and 1% Antibiotic-Antimycotic, and incubated for 24 hours in a constant temperature chamber at 37 °C with 5 % CO₂. Subsequently, the cells were treated with the compound prepared in Embodiment 1 and incubated for an additional 24 hours.

**[Table 1]**

| Category | Cell Viability |
|---|---|
| Untreated | 100% |
| Embodiment 1 Compound 5 uM | 95.9% |
| Embodiment 1 Compound 10 uM | 118.9% |

Referring to Table 1 and FIG. 2 above, the compound prepared in Embodiment 1 (represented by Chemical Formula 1) did not show toxicity to Keratinocyte (HaCaT) cells at concentrations of 5 and 10 uM, and moreover, at 10 uM treatment, cell viability increased, thus, confirming that it is a safe substance with low potential to cause skin irritation.

### Experimental Example 2

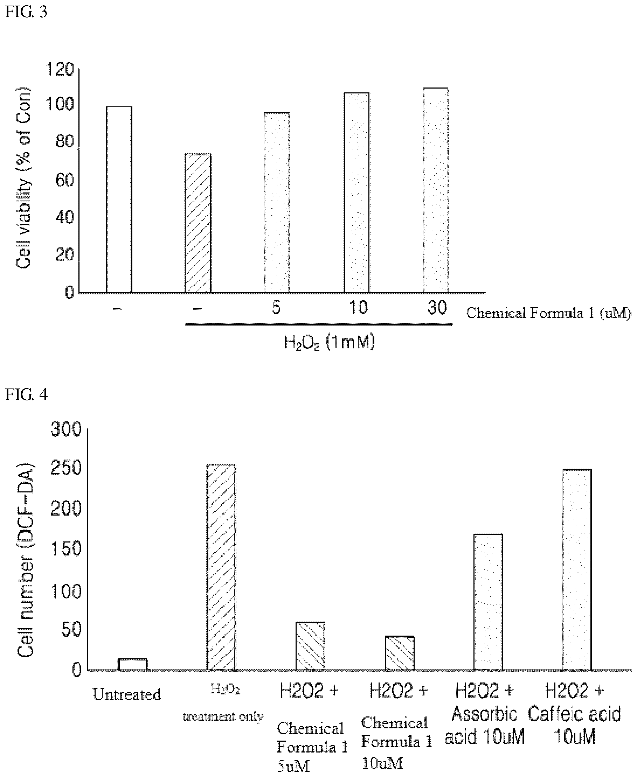

To examine the antioxidant effect of the compound prepared in Embodiment 1 against H₂O₂ oxidative toxicity, changes in cell viability due to H₂O₂ were measured, and the results are shown in Table 2 and FIG. 3 below. In the experiment, HaCaT cells were seeded in a 96-well plate and incubated for 24 hours under cell incubation condition. Subsequently, the cells were treated with 1 mM H₂O₂, and the control group and the compound prepared in Embodiment 1 were treated to the cells and further incubated for 12-16 hours. To investigate whether the compound protects skin cells from damage caused by H₂O₂-induced oxidative toxicity, cell viability was measured using a WST-1 assay.

**[Table 2]**

| Category | Number of fluorescent cells (Cell number) |
|---|---|
| Untreated | 15 |
| H₂O₂ treatment only | 255 |
| H₂O₂ + Ascorbic acid 10 uM | 170 |
| H₂O₂ + Caffeic acid 10 uM | 250 |
| H₂O₂ + Embodiment 1 Compound 5 uM | 60 |
| H₂O₂ + Embodiment 1 Compound 10 uM | 42 |

Referring to Table 2 and FIG. 3 above, it was confirmed that the increase in reactive oxygen species caused by H₂O₂ treatment was significantly reduced in cells treated with various concentrations of the compound prepared in Embodiment 1 (represented by Chemical Formula 1) compared to the positive control group (H₂O₂ treatment).

### Experimental Example 3

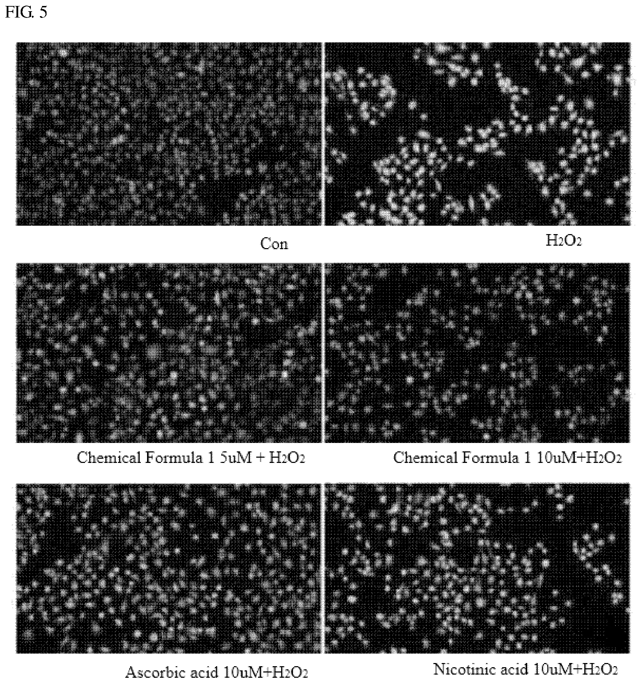

To investigate the inhibitory effect of the compound prepared in Embodiment 1 on H₂O₂-induced reactive oxygen species generation, DCF-DA staining was performed, and the results are shown in Table 3, FIG. 4, and FIG. 5 below. In the experiment, H₂O₂ cells were seeded at a density of 2×10⁵ cells per dish in a 35pi dish and incubated for 24 hours. Subsequently, the cells were treated with the compound prepared in Embodiment 1 and further incubated for 24 hours. After 10 minutes of loading with 5 mM DCF-DA, the cells were examined under a fluorescence microscope, and the number of DCF-DA fluorescent cells was counted using Image J.

**[Table 3]**

| Category | Number of fluorescent cells (Cell number) |
|---|---|
| Untreated | 15 |
| H₂O₂ treatment only | 255 |
| H₂O₂ + Ascorbic acid 10uM | 170 |
| H₂O₂ + Caffeic acid 10uM | 250 |
| H₂O₂ + Chemical Formula 1 5uM | 60 |
| H₂O₂ + Chemical Formula 1 10uM | 42 |

Referring to Table 3 and FIG. 4 above, it was confirmed that the increase in reactive oxygen species caused by H₂O₂ treatment was significantly reduced in cells treated with various concentrations of the compound prepared in Embodiment 1 (represented by Chemical Formula 1) compared to the positive control group (H₂O₂ treatment).

As described above, the novel compound represented by Chemical Formula 1 according to the present invention has excellent antioxidant activity without significant cytotoxicity, and thus may be used for various purposes in food, pharmaceuticals, and other chemical products.

While the preferred embodiments of the present invention have been described in detail above, the scope of the present invention is not limited thereto, and various modifications and improvements by the person skilled in the art using the basic concept of the present invention as defined in the following claims also fall within the scope of the present invention.

### INDUSTRIAL APPLICABILITY

The present invention may provide novel compounds, pharmaceutically or cosmetologically acceptable salts thereof, which exhibit superior antioxidant effects and may be applied in pharmaceutical compositions or cosmetic compositions.

## Claims

1. A compound represented by the following Chemical Formula 1, an isomer thereof, or a pharmaceutically acceptable salt thereof: 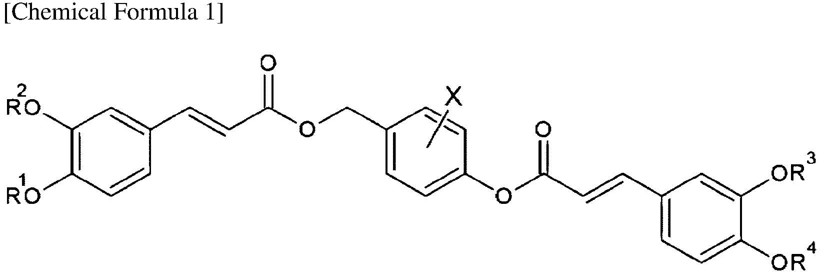
In the formula,
R¹ to R⁴ are each independently hydrogen, C₁-C₁₂ alkyl, acyl, or cyclic alkyl; and
X is hydrogen, fluoro, chloro, methyl, or methoxy.

2. The compound of claim 1, wherein the compound represented by Chemical Formula 1, isomer thereof, or pharmaceutically acceptable salt thereof are selected from a group consisting of compounds having the following structures.

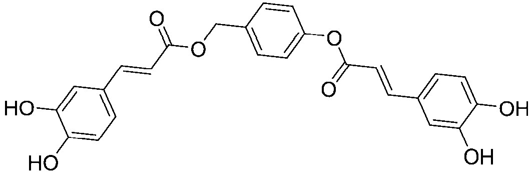

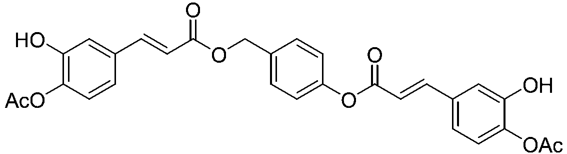

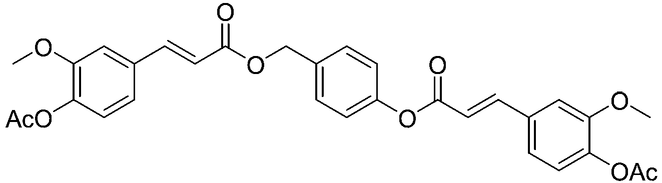

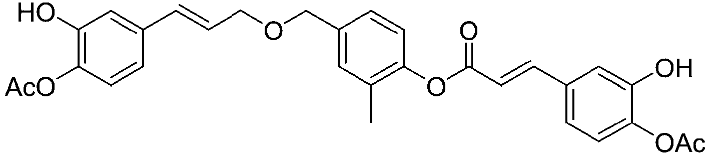

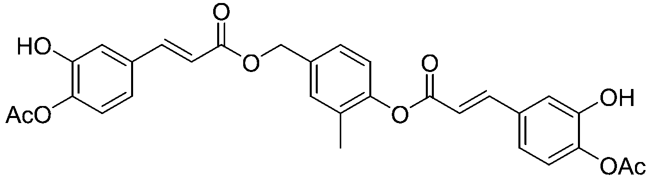

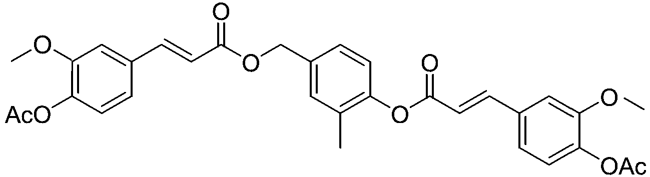

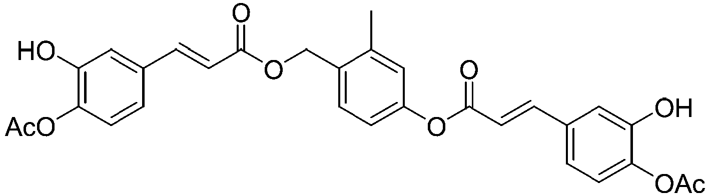

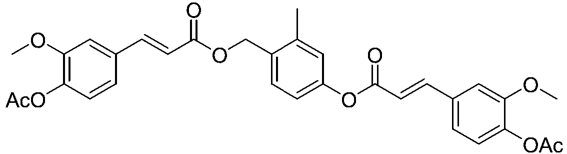

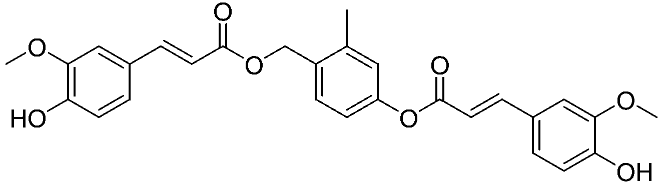

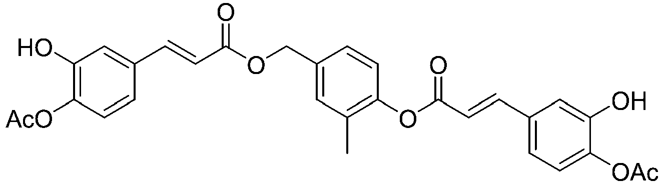

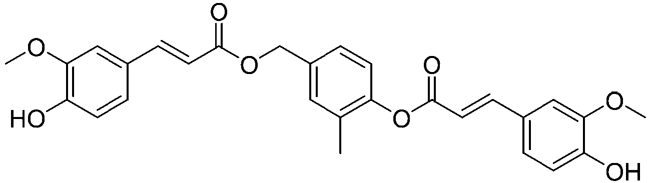

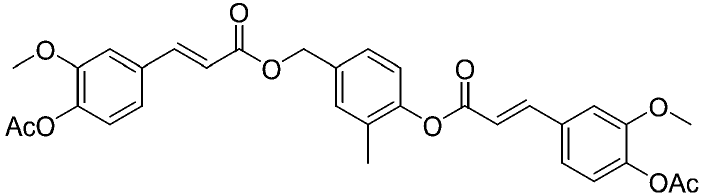

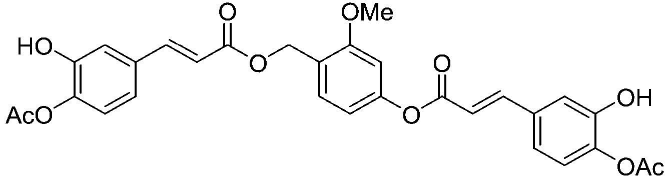

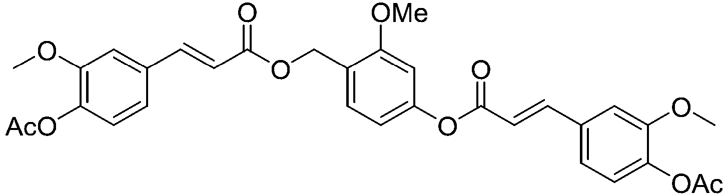

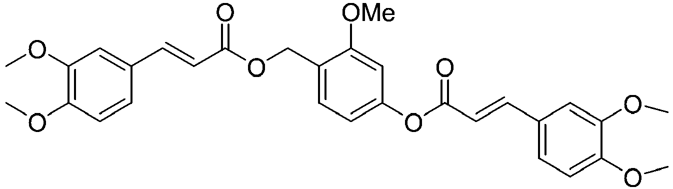

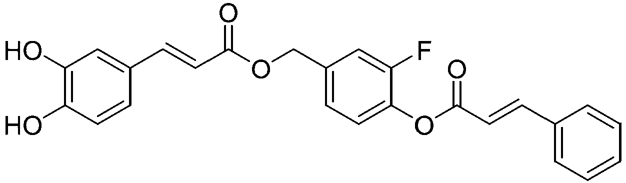

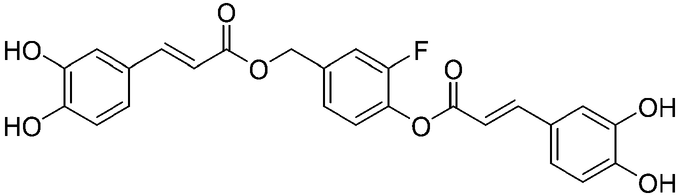

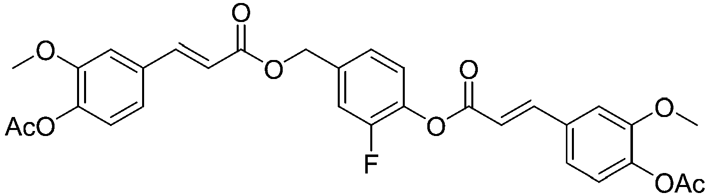

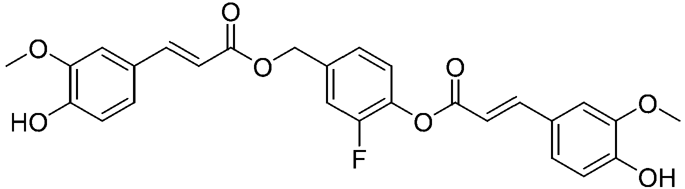

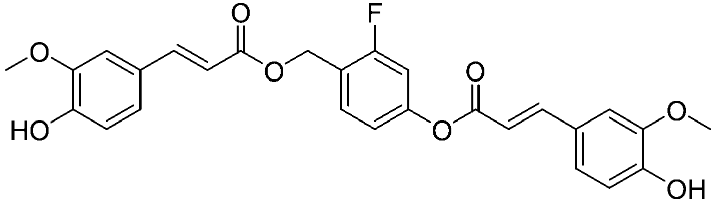

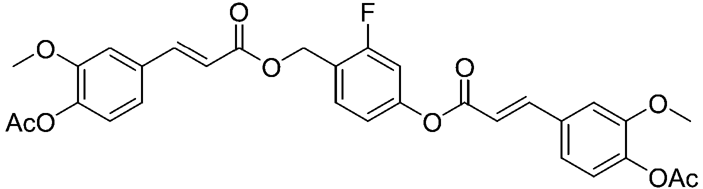

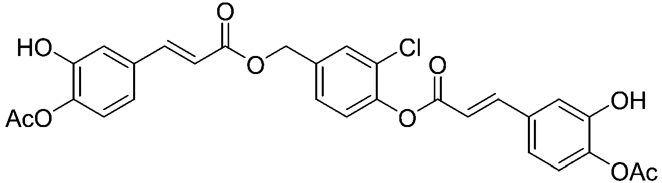

3. A pharmaceutical composition comprising:
a compound represented by the following Chemical Formula 1, an isomer thereof, or a pharmaceutically acceptable salt thereof as an active ingredient. 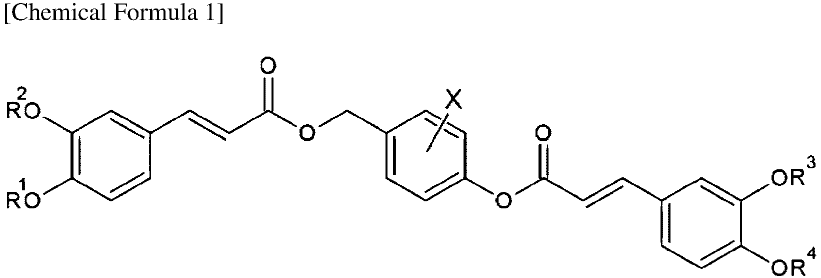
In the formula,
R¹ to R⁴ are each independently hydrogen, C₁-C₁₂ alkyl, acyl, or cyclic alkyl; and
X is hydrogen, fluoro, chloro, methyl, or methoxy.

4. A cosmetic composition comprising:
a compound represented by the following Chemical Formula 1, an isomer thereof, or a cosmetologically acceptable salt thereof as an active ingredient. 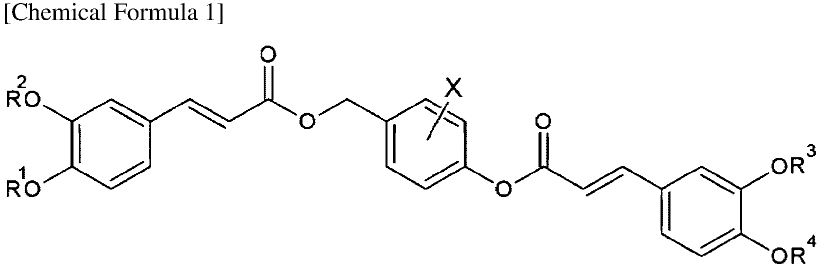
In the formula,
R¹ to R⁴ are each independently hydrogen, C₁-C₁₂ alkyl, acyl, or cyclic alkyl; and
X is hydrogen, fluoro, chloro, methyl, or methoxy.
